# EUROPÄISCHE PATENTANMELDUNG

(11) **EP 3 058 905 A1**
(43) Veröffentlichungstag der Anmeldung: **24.08.2016**
(21) Anmeldenummer: 15179062.3
(22) Anmeldetag: 30.07.2015
(51) Int. Cl.: A61F 9/04

(54) **AUGENKLAPPE UND VERFAHREN ZUR ERMITTLUNG VON BEWEGUNGS- UND/ODER LAGEDATEN UNTER VERWENDUNG DER AUGENKLAPPE**

(30) Priorität: 19.02.2015 DE 102015102373
(71) Anmelder: Prahs, Philipp, 93049 Regensburg (DE)
(72) Erfinder: Prahs, Philipp, 93049 Regensburg (DE)
(74) Vertreter: Benninger, Johannes

(57) **Zusammenfassung**

Es wird eine Augenklappe (10) zur zumindest teilweisen Abdeckung eines Auges und zu dessen Abschirmung vor mechanischen, klimatischen Einflüssen und/oder vor elektromagnetischer Strahlung vorgeschlagen. Die Augenklappe (10) umfasst eine das Auge und dessen Umgebung abdeckende Schale (12) aus biegesteifem Material sowie eine Sensoreinheit (14), die einen Lage- und/oder Bewegungssensor (16) zur kontinuierlichen Erfassung von Bewegungs- und/oder Lagedaten der Augenklappe (10), einen Mikrocontroller (18) zur Verarbeitung der Ausgangssignale (20) des Lage- und/oder Bewegungssensors (16), eine zur drahtlosen, bidirektionalen Kommunikation ausgebildete Datenschnittstelle (22) sowie einen elektrischen Energiespeicher (24) zur elektrischen Energieversorgung zumindest des Mikrocontrollers (18) und der Datenschnittstelle (22) umfasst.

Weiterhin wird ein Verfahren zur kontinuierlichen Ermittlung von Bewegungs- und/oder Lagedaten eines Kopfes mittels einer solchen Augenklappe (10) und einer Datenverarbeitungseinrichtung (30) vorgeschlagen. Die Datenverarbeitungseinrichtung (30) umfasst wenigstens eine Recheneinheit (34) zur Datenverarbeitung, einen Bildschirm (36) zur Visualisierung der verarbeiteten Daten (32) und eine zur drahtlosen, bidirektionalen Kommunikation ausgebildete Datenschnittstelle (38). Das Verfahren weist wenigstens die folgenden Schritte auf: Positionieren der Augenklappe (10) und der Sensoreinrichtung (14) am Auge; Aktivieren der Sensoreinrichtung (14); Übertragung der von der Sensoreinrichtung (14) ermittelten Bewegungs- und/oder Lagedaten an die Datenverarbeitungseinrichtung (30); Auswertung der übertragenen Bewegungs- und/oder Lagedaten in der Datenverarbeitungseinrichtung (30); Visualisierung der ausgewerteten Bewegungs- und/oder Lagedaten.

## Beschreibung

Die vorliegende Erfindung betrifft eine Augenklappe mit einem Lage- und/oder Bewegungssensor mit den Merkmalen des unabhängigen Anspruchs 1 sowie ein Verfahren zur kontinuierlichen Ermittlung von Bewegungs- und/oder Lagedaten unter Verwendung der Augenklappe gemäß unabhängigem Verfahrensanspruch 9.

In der Augenheilkunde gibt es eine Vielzahl von Situationen, in welchen eine stabile, möglichst nicht oder nur wenig veränderliche Positionierung eines Kopfes angezeigt ist. Als Beispiel kann hierbei der Einsatz einer sogenannten intraokularen Gastamponade dienen, welche nach operativen Eingriffen am Augapfel, bspw. bei einer Netzhautablösung, Verwendung findet, indem die im Augapfel vorhandene Substanz, der Glaskörper, wenigstens teilweise durch eine Luft- oder Gasfüllung ersetzt wird.

Ein weiteres Beispiel stellen lamelläre Hornhauttransplantationen dar, bei der eine Luftfüllung in der Vorderkammer des Auges verbleibt und das Hornhauttransplantat zur Adhäsion an der Empfängerhornhaut bringt.

Eine solche Gastamponade kann einerseits das Anlegen der Netzhaut an die Innenwand eines Augapfels unterstützen und andererseits dazu dienen, einen Flüssigkeitsdurchtritt durch kleine Löcher o.ä. in der Netzhaut zu verhindern, indem durch die an der Gas/Wassergrenze vorliegende Oberflächenspannung eine durch Kapillareffekte, etc. ausgelöste Flüssigkeitsströmung durch Netzhautlöcher oder ähnliche Verletzungen unterbunden wird. Die Gastamponade selbst muss nicht durch weitere Maßnahmen entfernt werden, das darin enthaltene Gas, meist Schwefelhexafluorid, baut sich im Verlauf des Heilungsprozesses durch resorptive Prozesse von selbst ab, wobei der Durchmesser der in den Augapfel eingebrachten Gasblase stetig geringer wird.

Es ist nun verständlich, dass die Gastamponade während des Heilungsverlaufs, insbesondere bei abnehmendem Durchmesser der Gasblase, möglichst unveränderlich an der dafür vorgesehenen Stelle, z.B. der Operationsstelle, gehalten werden sollte. Die für diesen Fall optimale Lage des Auges, und damit des Kopfes, u.U. auch des gesamten Körpers, kann zwar von einem Augenarzt vorgegeben werden. Allerdings ist es für den Träger der Augenklappe nach einer Lageänderung, die sich bspw. während des Schlafes auch ungewollt ereignen kann, nicht oder nur schwer möglich, wieder in den optimalen Zustand zurückzukehren, so dass ein Hilfsmittel, welches dies ermöglicht, von großem Nutzen wäre.

Die DE 698 16 318 T2 beschreibt einen Verband in Form einer Augenklappe, bestehend aus einer Kompresse, einem Mikrocontroller und einem Sensor. Der Zweck der beschriebenen Sensoranordnung liegt in der Überwachung der Tragezeiten des Verbands, wobei der in der Kompresse integrierte Sensor vorhandenen bzw. fehlenden Hautkontakt an den Mikrocontroller meldet, welcher die Daten speichert. Über eine Datenschnittstelle können diese dann ausgelesen werden.

Die DE 10 2010 038 028 A1 offenbart ein Verfahren zur Erfassung der Gemütszustände eines Pferdes, bei welchem ein Bewegungssensor o.ä. bspw. die Ohrstellung erfasst, diese in einer elektronischen Einheit speichert und die gespeicherten Daten über eine Schnittstelle zur Auswertung bereitstellt.

Die WO 2010/038156 A1 betrifft ein drahtloses EKG-Monitoringsystem zur ambulanten Patientenüberwachung. Die von einer entsprechenden Sensorik registrierten Daten werden digitalisiert und an einen Smartphone zur Auswertung mittels einer geeigneten Software weitergegeben. Die Software ist weiterhin dazu geeignet, Statusinformationen an entfernte Stellen zu übertragen.

Die WO 2011/035262 A1 zeigt ein Verfahren zur Einsetzung eines implantierbaren MEMS-Sensors in der Augenheilkunde, wobei der Sensor zur Messung des Augeninnendrucks ausgebildet ist.

Angesichts der aus dem Stand der Technik bekannten Maßnahmen besteht eine vorrangige Aufgabe der Erfindung darin, eine Vorrichtung und ein Verfahren bereitzustellen, mit welchen sich die Position des Auges bzw. des Kopfes möglichst exakt erfassen lässt, welche es dem Augenarzt ermöglichen, eine optimale Sollposition vorzugeben und welche es dem Träger der Augenklappe durch optische, akustische oder eine anders geartete Rückmeldung ermöglichen, die genannte Sollposition nach einer Lageänderung wieder einzunehmen.

Weiterhin ist es Aufgabe der Erfindung, durch die Bereitstellung der Möglichkeit einer Aufzeichnung und Auswertung der Bewegungs- und/oder Lagedaten die Voraussetzungen zu schaffen, Heilerfolge oder deren Chancen auf Basis statistischer Daten einschätzen zu können.

Schließlich ist es Aufgabe der erfindungsgemäßen Augenklappe, neben der Möglichkeit der Datenerfassung auch eine Schutzfunktion für das unter der Augenklappe verborgene Auge bereitzustellen.

Die genannten Aufgaben werden jeweils gelöst mit den Gegenständen der unabhängigen Ansprüche. Weitere vorteilhafte Ausgestaltungen werden in den Unteransprüchen beschrieben. Zur Lösung der genannten Aufgaben schlägt die vorliegende Erfindung eine Augenklappe zur zumindest teilweisen oder partiellen Abdeckung eines menschlichen oder tierischen Auges und zu dessen Abschirmung vor mechanischen und/oder klimatischen Einflüssen und/oder vor elektromagnetischer Strahlung, insbesondere vor Lichtstrahlung vor.

Wenn in diesem Zusammenhang von einem Auge die Rede ist, so ist damit in aller Regel ein menschliches Auge gemeint, das beispielsweise einer Heilbehandlung oder Therapie unterzogen werden kann oder das sich in Rekonvaleszenz befindet. Allerdings ist es nicht ausgeschlossen, die erfindungsgemäße Augenklappe sowie das erfindungsgemäße Verfahren auch für Tiere zu verwenden bzw. anzuwenden, da es grundsätzlich denkbar ist, auch bei Tieren eine Heilbehandlung an einem Auge durchzuführen und dieselben Daten zu erheben wie dies bei einem menschlichen Auge der Fall wäre.

Wenn zudem von einer zumindest teilweisen oder partiellen Abdeckung des Auges die Rede ist, so ist dies dahingehend zu verstehen, dass die Lageerfassung und der Einsatz der Augenklappe auch dann funktionieren kann, wenn das Auge selbst nicht vollständig abgedeckt oder von den genannten klimatischen, mechanischen und/oder elektromagnetischen Einflüssen abgeschirmt ist. Wenn die Augenklappe auch im Normalfall das Auge weitgehend bzw. vollständig abdeckt, ist damit nicht ausgeschlossen, dass in Einzelfällen das Auge nur partiell abgedeckt sein kann. Es sein betont, dass ein solcher Fall nicht die Einsatzmöglichkeit der erfindungsgemäßen Augenklappe oder die Effektivität des erfindungsgemäßen Verfahrens schmälert.

Die erfindungsgemäße Augenklappe weist wenigstens eine das Auge und dessen Umgebung abdeckende Schale aus biegesteifem Material sowie eine Sensoreinheit oder ein Sensormodul auf, die/das zumindest einen Lage- und/oder Bewegungssensor zur kontinuierlichen Erfassung von Bewegungs- und/oder Lagedaten der Augenklappe, einen Mikrocontroller zur Verarbeitung der Ausgangssignale des wenigstens einen Lage- und/oder Bewegungssensors, eine zur drahtlosen, bidirektionalen Kommunikation ausgebildete Datenschnittstelle sowie einen elektrischen Energiespeicher zur elektrischen Energieversorgung zumindest des Mikrocontrollers und der Datenschnittstelle umfasst. Selbstverständlich können auch zwei oder mehr Sensoreinheiten bzw. Sensormodule vorhanden sein, deren Daten vom Mikrokontroller verarbeitet werden. Der Sensoreinheit bzw. den Sensoreinheiten kann außerdem eine dauerhafte und/oder temporär mit dem Lage- und/oder Bewegungssensor koppelbare Datenspeichereinheit zur Speicherung der vom Sensor bzw. von den Sensoren erfassten Bewegungs- und/oder Lagedaten zugeordnet sein. In der erfindungsgemäßen Augenklappe kann die Sensoreinrichtung bzw. die Sensoreinheit (auch: die Mehrzahl von Sensoreinrichtungen bzw. Sensoreinheiten) angebracht bzw. baulich integriert sein; die Sensoreinheit, welche an die übliche Kontur solcher Augenklappen angepasst ist, dient der Ermittlung der Bewegungs- und/oder Lagedaten und/oder der Speicherung bzw. Übermittlung der ermittelten Daten, was wiederum den gewünschten Heilungsprozess bei den oben genannten Augenoperationen unterstützen kann. Die solcherart ausgestattete Augenklappe wird anstelle der üblicherweise für diesen Zweck eingesetzten Augenklappe verwendet und ist somit in der Lage, nicht nur die ihr zugewiesene Funktion als schützende Abdeckung des Auges gegenüber mechanischen Beeinträchtigungen wie Berührungen, Verschmutzungen etc. zu erfüllen sondern darüber hinaus den Heilungserfolg auch durch Sensorunterstützung und durch Unterstützung von akustischen oder anderen elektronischen Hilfsmitteln zu unterstützen. Je nach Ausgestaltung, d.h. Größe und Form der Sensoreinrichtung, kann es auch möglich sein, eine standardgemäße Augenklappe zusätzlich mit der Sensoreinrichtung zu versehen, die in diesem Fall außen an der Augenklappe befestigt sein kann, wahlweise auch mittels lösbarer Befestigungseinrichtungen.

Bei einer Variante der erfindungsgemäßen Augenklappe können der Sensoreinheit Mittel zur Ausgabe eines akustischen Signals zugeordnet sein, wodurch dem Träger der Augenklappe signalisiert werden kann, dass er eine Lageänderung vornehmen sollte, um das Auge wieder in eine günstigere Position zu bringen.

Weiterhin kann es sinnvoll sein, wenn der erwähnte Mikrocontroller eine Signalquelle zur Erzeugung eines absoluten oder relativen Zeitindex enthält. Vorzugsweise sind die Daten zum Zeitindex und/oder die Bewegungs- und/oder Lagedaten in der Datenspeichereinheit speicherbar und/oder über die Datenschnittstelle übertragbar.

Durch bzw. über die zur drahtlosen, bidirektionalen Kommunikation ausgebildete Datenschnittstelle kann wahlweise ein externer Zugriff auf die Sensoreinrichtung ermöglicht werden. Diese zur drahtlosen, bidirektionalen Kommunikation ausgebildete Datenschnittstelle kann bspw. mit dem sog. Bluetooth-Standard arbeiten.

Eine weitere Variante der Augenklappe kann vorsehen, dass der temporär mit der Sensoreinrichtung verbindbaren Datenspeichereinheit durch eine MicroSD-Karte gebildet ist.

Bei der erfindungsgemäßen Augenklappe können bspw. wenigstens zwei der Bestandteile der Sensoreinheit in einem Bauteil integriert sein. Wahlweise kann die Augenklappe durch zwei Schalen gebildet sein, zwischen denen wenigstens eines der Bestandteile der Sensoreinheit angeordnet ist.

Erfindungsgemäß besteht die Augenklappe aus mindestens einer Schale, welcher der Form üblicherweise für diesen Zweck eingesetzter Augenklappen weitgehend oder vollständig entspricht. An ihrer Außenseite sind wenigstens ein Lage- und/oder Bewegungssensor, bspw. ein Inertialsensor, ein Mikrocontroller, ein Energiespeicher und eine Datenschnittstelle derart angebracht, dass eine Beeinträchtigung der Schutzfunktion der Augenklappe verhindert wird. Der mindestens eine Sensor bzw. Inertialsensor, vorzugsweise ein Halbleiterbaustein in MEMS-Technologie, dient der Messung von Beschleunigungs- und Drehbewegungen im Raum; die solcherart ermittelten Daten sind infolgedessen in der Lage, jedwede Änderung der Position des Sensors hinsichtlich eines vorhandenen bzw. vorgegebenen Koordinatensystems aufzuzeigen. Es ist dabei weitgehend unerheblich, auf welche Art das Koordinatensystem normiert ist, ob also z.B. der Gravitationsvektor als maßgebliche Einflussgröße verwendet wird; die Lage der Achsen eines solchen Koordinatensystems ist grundsätzlich frei bestimmbar. Die tatsächliche Lage des Auges bzw. Kopfes lässt sich nun über die ermittelten Bewegungsdaten als Abweichung zu den zuvor erfassten Bewegungen definieren.

Der Mikrocontroller beinhaltet neben einem Prozessorkern wenigstens einen Arbeits- und Programmspeicher zur Ausführung von Programmcode und stellt weiterhin eine Anzahl von Funktionen zur Anbindung externer Komponenten wie dem Inertialsensor, wenigstens einer Datenschnittstelle u.ä. bereit. Auf diese Weise ist es möglich, grundsätzliche Abläufe der Messdatenermittlung, also der Ermittlung der Bewegungs- und/oder Lagedaten durch Auslesen der vom Inertialsensor bereitgestellten Werte, bereits in der Sensoreinrichtung durchzuführen und die solcherart bestimmten Daten zur späteren Datenverarbeitung weiterzuleiten und/oder zu speichern.

Die schnell fortschreitende technische Entwicklung erlaubt dabei z.B. durch Erhöhung der Speicherkapazität und der Rechenleistung eines solchen Mikrocontrollers, dass immer komplexere Aufgaben, wie Datenermittlung, Bewertung, Speicherung und evtl. dadurch bedingte Folgeaktionen durch den Mikrocontroller selbst erledigt werden können, welcher zu diesem Zweck selbstverständlich auf die Kommunikation mit einer externen Datenverarbeitungseinrichtung angewiesen ist, über welche einerseits der entsprechende Programmcode, etc. über eine Datenschnittstelle in den Mikrocontroller gelangt und über welche andererseits die ermittelten und/oder entsprechend aufbereiteten Daten mittels der Datenschnittstelle in einen Datenspeicher und/oder über eine drahtlose Verbindung an eine Datenverarbeitungseinrichtung weitergegeben werden.

Sehr hilfreich für die Auswertung der Daten kann eine Ausführungsform der Erfindung sein, bei welcher, z.B. durch den Mikrocontroller, ein Zeitindex generiert wird, welcher es erlaubt, die vom Inertialsensor zur Verfügung gestellten und, je nach gewählter Ausgestaltung, vom Mikrocontroller aufbereiteten Bewegungs- und/oder Lagedaten in einen zeitlichen Kontext einzubetten. Es ist dabei weitgehend unerheblich, ob der Zeitindex absolut, d.h. mit realen Zeitangaben wie aktueller Uhrzeit o.ä. oder relativ, z.B. durch Festlegung eines willkürlichen Startzeitpunktes bereitgestellt wird, solange die Zuordnung der Bewegungs- und/oder Lagedaten zu einem zeitlichen Ablauf, eventuell auch zu äußeren Ereignissen wie bspw. Untersuchungen hergestellt werden kann.

Die Datenschnittstelle kann auf mehrere Arten ausgeführt sein; bevorzugt wird die Ausgestaltung als Funkmodul, welches es ermöglicht, die vom Inertialsensor erhaltenen Daten drahtlos an eine Datenverarbeitungseinrichtung zu senden, das für die Auswertung der gesendeten Daten sorgt. Idealerweise ist die Datenschnittstelle so ausgebildet, dass die Kommunikation bidirektional erfolgen kann, so dass Daten nicht nur gesendet sondern auch empfangen werden können. Dies kann z.B. eine Kalibrierung des Inertialsensors bzw. des Mikrocontrollers erlauben, wie im Weiteren noch ausgeführt werden wird.

Das entsprechende Funkmodul der Datenschnittstelle ist sinnvollerweise nicht für die Kommunikation über größere Entfernungen hin ausgelegt, sondern dient in erster Linie der Kurzstreckenübertragung. Bevorzugt wird ein Modul, welches nach dem Bluetooth-Standard arbeitet, wobei aber andere existierende oder zukünftige Standards ebenso infrage kommen können, solange sie den energetischen und sicherheitsrechtlichen Anforderungen genügen.

In Anwendungsfällen, in denen die direkte und gleichzeitige Verwendung einer Datenverarbeitungseinrichtung nicht möglich oder sinnvoll ist, kann es auch möglich sein, die Datenschnittstelle alternativ oder zusätzlich mit einer Datenspeichereinheit, bspw. einer handelsüblichen MicroSD-Karte auszustatten, welche zur Datenaufnahme mit der Datenschnittstelle temporär verbunden wird. Dies würde es bspw. ermöglichen, einen vollständigen Datensatz auch dann zu erhalten, wenn im Aufzeichnungszeitraum dauerhaft keine Verbindung zu einer Datenverarbeitungseinrichtung vorhanden ist oder für eine gewisse Zeit unterbrochen wurde. Zum Einlesen der auf der Datenspeichereinheit aufgezeichneten Daten kann diese im Anschluss von der Datenschnittstelle entfernt und an eine Datenverarbeitungseinrichtung angeschlossen werden.

Alternativ dazu kann auch vorgesehen sein, eine Datenspeichereinheit fest, d.h. dauerhaft an die Datenschnittstelle, und damit die Sensoreinrichtung anzubinden und die Daten auf diese Weise ergänzend zur Drahtlosübertragung in der Datenspeichereinheit der Sensoreinrichtung vorzuhalten. Das Auslesen der Daten aus der Datenspeichereinheit kann in einem solchen Fall bspw. drahtlos erfolgen, sobald eine Verbindung zu einer Datenverarbeitungseinrichtung hergestellt werden kann.

Die für den Betrieb der vorgenannten Bestandteile der Sensoreinrichtung benötigte elektrische Leistung muss von einem Energiespeicher zur Verfügung gestellt werden und richtet sich nach der Anzahl der zu versorgenden Komponenten, deren Auslegung, dem zur Verfügung stehenden Platz und sinnvollerweise auch dem Gewicht des Energiespeichers. Da die überwiegende Zahl der Bestandteile der Sensoreinrichtung auf Halbleitertechnologie beruht, welche infolgedessen ein eher geringes Gewicht aufweisen, ist das Gewicht des Energiespeichers von nicht geringer Bedeutung, bedeutet ein hohes Gesamtgewicht der erfindungsgemäßen Augenklappe doch nicht nur eine zusätzliche Belastung für den Träger der Augenklappe und, je nach Ausgestaltung, einen zusätzlichen Aufwand bei der Befestigung der Augenklappe, sondern kann auch den Heilungserfolg beeinflussen, wenn der Träger der Augenklappe z.B. aufgrund des hohen Auflagegewichts seine optimale Lagerungsposition stärker als nötig ändert.

Als vorteilhaft hat sich in diesem Zusammenhang daher die Verwendung einer handelsüblichen Knopfzelle erwiesen; diese sind in verschiedenen Leistungsstufen erhältlich, preiswert und weisen bei geringer Größe ein niedriges Gewicht auf.

In einer weiteren Ausführungsform der Augenklappe kann es angedacht sein, zusätzlich eine akustische Signalquelle auf der Augenklappe anzuordnen. Eine solche Signalquelle kann z.B. Hinweistöne einfacher Art erzeugen, bspw. bei Verlust der Kommunikationsverbindung zur Datenverarbeitungseinheit, oder auch komplexere akustische Ausgaben wie Sprachausgaben wiedergeben, sofern die Verarbeitungsleistung des Mikrocontrollers dies zulässt.

In einer alternativen Ausführungsform der erfindungsgemäßen Augenklappe kann es vorgesehen sein, dass zwei der Komponenten der Sensoreinrichtung, z.B. Inertialsensor und Mikrocontroller, in einem Bauteil integriert sind, bei entsprechender Ausgestaltung auch drei oder mehr Komponenten, so dass bspw. Inertialsensor, Mikrocontroller, Datenschnittstelle und gegebenenfalls Datenspeichereinheit in einem Bauteil vereint sind.

Eine weitere Ausführungsform der Augenklappe kann vorsehen, dass die Augenklappe aus zwei Schalen besteht, zwischen welchen wenigstens eine der Komponenten, d.h. der Bestandteile der Sensoreinrichtung, angebracht ist. Eine solche Ausführung schützt einerseits die jeweilige Komponente vor Beschädigungen und trägt andererseits dazu bei, sowohl den Tragekomfort für den Träger der Augenklappe zu erhöhen, indem bspw. die Anzahl der abstehenden Teile auf der Augenklappe verringert wird, als auch die Befestigung der Augenklappe am Auge zu erleichtern.

Bei höherer Integration der Komponenten kann es auch möglich sein, mehrere oder sogar alle der Komponenten zwischen den beiden Schalen einer solchen Augenklappe anzubringen.

Weiterhin sieht die Erfindung ein Verfahren zur kontinuierlichen Ermittlung von Bewegungs- und/oder Lagedaten eines Kopfes mittels der erfindungsgemäßen Augenklappe und einer Datenverarbeitungseinrichtung vor.

Die Augenklappe weist eine Sensoreinrichtung mit wenigstens einem Inertialsensor, einem Mikrocontroller, einem Energiespeicher und einer zur drahtlosen, bidirektionalen Kommunikation ausgebildeten Datenschnittstelle auf, die Datenverarbeitungseinrichtung beinhaltet wenigstens eine zur drahtlosen, bidirektionalen Kommunikation ausgebildete Datenschnittstelle, eine Recheneinheit zur Datenverarbeitung und einen Bildschirm zur Visualisierung der verarbeiteten Daten auf.

Das Verfahren sieht vor, eine Augenklappe mit der bereits beschriebenen Sensoreinrichtung am Auge zu befestigen, die Sensoreinrichtung zu aktivieren, Bewegungs- und/oder Lagedaten von der Sensoreinrichtung an die Datenverarbeitungseinrichtung zu übertragen, die übertragenen Daten in der Datenverarbeitungseinrichtung auszuwerten und zu visualisieren. Bei dem Verfahren kann die Sensoreinheit vorzugsweise mit einer dauerhaft und/oder temporär mit der Sensoreinheit verbindbaren Datenspeichereinheit kommunizieren, in welcher die Bewegungs- und/oder Lagedaten zusätzlich oder alternativ zur Datenübermittlung über die Datenschnittstelle gespeichert werden können. Außerdem kann die Sensoreinheit eine Signalquelle zur Erzeugung eines absoluten oder relativen Zeitindex enthalten, welcher zusammen mit den Bewegungs- und/oder Lagedaten an die Datenverarbeitungseinrichtung übermittelt und/oder in der Datenspeichereinheit gespeichert wird. Diese in der Datenspeichereinheit gespeicherten Daten können dann unabhängig von der zur drahtlosen, bidirektionalen Kommunikation ausgebildeten Datenschnittstelle an die Datenverarbeitungseinrichtung übermittelt werden. Wahlweise können die Bewegungs- und/oder Lagedaten innerhalb eines beliebigen Koordinatensystems auf wenigstens einen frei bestimmbaren Sollwert normiert werden. Auch kann vorgesehen sein, dass jede Abweichung der Bewegungs- und/oder Lagedaten vom wenigstens einen Sollwert wenigstens eine Aktion auslösen kann. Als sinnvolle Variante hat sich dabei herausgestellt, dass es sich bei der wenigstens einen Aktion um einen visuellen und/oder akustischen und/oder durch wenigstens ein Vibrationssignal dargestellten Hinweis handeln kann. Allerdings sind auch andere Signalvarianten denkbar, die den gewünschten Zweck ebenso erfüllen können.

Das Verfahren sieht weiterhin eine Kalibrierung des Sensors zur eindeutigen Bestimmung der Lage des Kopfes vor, wobei die Sensoreinheit in einer bestimmten Orientierung angeordnet ist. Die Kalibrierung erfolgt nach Anlegen der Augenklappe und durch Bestimmung der Lage der Körperachse in Relation zur Sensoreinrichtung, bei welcher die Position des Kopfes eindeutig bestimmt werden kann.

Mit dieser Kalibrierung kann die Sensorposition auf das beabsichtigte Referenzsystem abgestimmt werden. Da die Anatomie der Knochen um das menschliche Auge variabel ist, kann der Sensor nach dem Aufkleben bei jedem Patienten eine individuelle Orientierung zum Kopf einnehmen. Mögliche Probleme aus diesen Abweichungen können dadurch gelöst werden, dass die Sensororientierung direkt nach der Operation im Liegen registriert wird. Aus operationstechnischen Gründen besteht hier eine flache Rückenlage; zur Kontrolle kann bspw. der Rotlichtreflex des senkrecht stehenden Operationsmikroskops dienen. Außerdem braucht es zur vollständigen Definition der Sensororientierung noch die Lage der Körperachse in Relation zum Sensor; dies kann durch Aufsitzen lassen des Patienten (z.B. mit Hilfe der Operationsliege) erreicht werden. Mit dieser zweiten Orientierung ist dann die Lage des Sensors zum Kopf bestimmt, genau genommen überbestimmt und es kann noch ein Maß für die Güte der Kalibrierung angegeben werden, falls z.B. die Rotationsachse des "Aufsitzenlassens" nicht direkt in der Horizontalen liegt.

Zur Anleitung/ Überwachung und Aufzeichnung der heilungsrelevanten postoperativen Kopfhaltung eines Patienten schlägt die Erfindung vor, eine herkömmliche, feste Augenklappe (die ohnehin getragen werden muss) mit einem Inertialsensor sowie einem Mikroprozessor mit Funkmodul zu versehen. Zur Stromversorgung dieser Komponenten soll zudem eine Knopfzelle bzw. Lithiumbatterie o. dgl. angebracht werden. Der Inertialsensor, bestehend aus einem Dreiachsen-Beschleunigungsmesser sowie einem Dreiachsen-Gyroskop misst die Bewegung und Lagerung des Patienten. Die gemessenen Daten werden von dem Mikroprozessor verarbeitet und per Funk (z.B. Bluetooth) an ein Datenerfassungsgerät, beispielsweise an ein Smartphone, ein Tablet oder einen Computer gesendet. Eine speziell programmierte Software kann mittels optischer/ akustischer oder Vibrationssignale die optimale Kopforientierung des Patienten anleiten und/oder anhand der empfangenen Daten bei einer Abweichung von dieser Position eine Warnung ausgeben. Gleichzeitig lassen sich die Sensordaten auch aufzeichnen und im Rahmen von klinischen Studien oder zur Qualitätskontrolle des Heilungsverlaufes auswerten. Die Anwendungsmöglichkeit der Erfindung liegt hauptsächlich in der postoperativen Behandlung bestimmter Augenoperationen (z. B. pars-plana-Vitrektomie bei Netzhautablösung und Makulaforamina oder lamellären Hornhauttransplantationen), bei welchen die Lagerung des Kopfes für den Heilungsverlauf von Bedeutung ist.

Wie oben bereits erwähnt, kann die Augenklappe wenigstens eine Sensoreinheit bzw. ein Sensormodul aufweisen. Diese Formulierung ist generell dahingehend zu verstehen, dass anstelle der erwähnten Sensoreinheit bzw. dem erwähnten Sensormodul wahlweise auch zwei oder mehr Sensoreinheiten bzw. Sensormodule vorhanden sein können, deren Daten vom Mikrokontroller verarbeitet werden. Wenn also an irgendeiner Stelle der vorstehenden Beschreibung oder auch der nachfolgenden Figurenbeschreibung, die auf ein Ausführungsbeispiel Bezug nimmt, von einer Sensoreinheit, einer Sensoreinrichtung oder einem Sensormodul die Rede ist, so umfasst diese Formulierung jeweils auch die Option mehrerer Sensoreinheiten, mehrerer Sensoreinrichtungen oder mehrerer Sensormodule, ohne dass dies jeweils gesondert erwähnt sein muss.

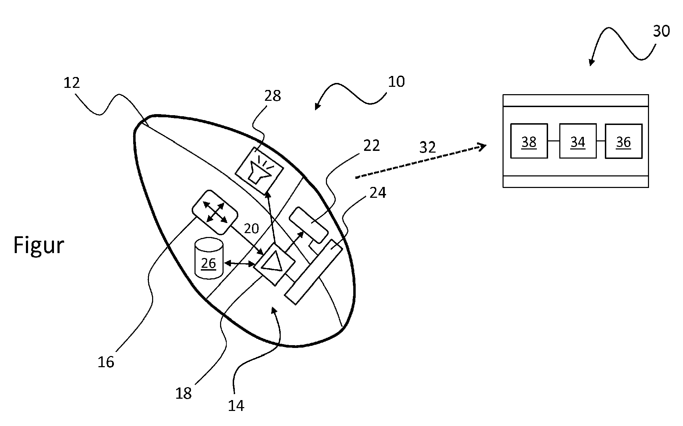

Im Folgenden soll ein Ausführungsbeispiel die Erfindung und ihre Vorteile anhand der beigefügten einzigen Figur näher erläutern. Die Größenverhältnisse der einzelnen Elemente zueinander in der Figur entsprechen nicht immer den realen Größenverhältnissen, da einige Formen vereinfacht und andere Formen zur besseren Veranschaulichung vergrößert im Verhältnis zu anderen Elementen dargestellt sind.

Die Figur zeigt eine schematische Perspektivansicht einer Ausführungsvariante einer erfindungsgemäßen Augenklappe mit den darin integrierten zusammenwirkenden Komponenten der Sensoreinheit.

Die dargestellte Ausführungsform stellt lediglich ein Beispiel dar, wie die erfindungsgemäße Vorrichtung ausgestaltet sein kann, soll jedoch nicht als abschließende Begrenzung verstanden werden.

Die schematische Perspektivansicht der einzigen Figur zeigt eine Ausführungsvariante einer erfindungsgemäßen Augenklappe 10, die der Abdeckung eines menschlichen (oder auch tierischen) Auges und dessen Abschirmung vor mechanischen, klimatischen Einflüssen und/oder vor elektromagnetischer Strahlung, insbesondere von Lichtstrahlung dient. Nicht zuletzt jedoch dient die erfindungsgemäße Augenklappe 10 aufgrund ihrer Ausstattung der Unterstützung von Heilungsmaßnahmen, indem insbesondere eine gewünschte Orientierung des Auges im Raum erfasst und Abweichungen von einer vorgegebenen Orientierung erkannt bzw. ggf. auch verhindert werden können. Die Augenklappe 10 weist eine das Auge und dessen Umgebung partiell, insbesondere vollständig abdeckende Schale 12 aus biegesteifem Material sowie eine Sensoreinheit 14 auf, die zumindest wenigstens einen Lage- und/oder Bewegungssensor bzw. einen sog. Inertialsensor 16 zur kontinuierlichen Erfassung von Bewegungs- und/oder Lagedaten der Augenklappe 10, einen Mikrocontroller 18 zur Verarbeitung der Ausgangssignale 20 des wenigstens einen Lage- und/oder Bewegungs- bzw. Inertialsensors 16, eine zur drahtlosen, bidirektionalen Kommunikation ausgebildete Datenschnittstelle 22 sowie einen elektrischen Energiespeicher 24 zur elektrischen Energieversorgung zumindest des Mikrocontrollers 18 und der Datenschnittstelle 22 umfasst.

Weiterhin umfasst die Augenklappe 10 eine der Sensoreinheit 14 zugeordnete Datenspeichereinheit 26, die dauerhafte und/oder temporär mit dem Lage- und/oder Bewegungssensor 16 gekoppelt sein kann, und die der Speicherung der vom Sensor 16 erfassten Bewegungs- und/oder Lagedaten dient. Die Sensoreinheit 14 enthält weiterhin Mittel 28 zur Ausgabe wenigstens eines akustischen Signals, bspw. einen Lautsprecher oder dergleichen. Wahlweise kann der Mikrocontroller 18 eine Signalquelle zur Erzeugung eines absoluten oder relativen Zeitindex aufweisen. Dieser Zeitindex und/oder die Bewegungs- und/oder Lagedaten können in der Datenspeichereinheit 26 gespeichert und wahlweise auch über die Datenschnittstelle 22 übertragen werden.

Über die zur drahtlosen, bidirektionalen Kommunikation ausgebildete Datenschnittstelle 22 kann wahlweise ein externer Zugriff auf die Sensoreinheit 14 ermöglicht werden. Die zur drahtlosen, bidirektionalen Kommunikation ausgebildete Datenschnittstelle 22 kann insbesondere mit dem Bluetooth-Standard arbeiten.

Bei der temporär mit der Sensoreinrichtung 14 verbindbaren Datenspeichereinheit 26 kann es sich bspw. um eine MicroSD-Karte handeln.

Bei der Augenklappe können vorzugsweise wenigstens zwei der Bestandteile der Sensoreinheit 14 in einem Bauteil integriert sein.

Bei einer alternativen Variante der Augenklappe kann diese ggf. durch zwei Schalen gebildet sein, zwischen denen wenigstens eines der Bestandteile der Sensoreinheit 14 angeordnet ist.

Mit der vorliegenden Erfindung wird ein Verfahren zur kontinuierlichen Ermittlung von Bewegungs- und/oder Lagedaten eines menschlichen Kopfes mittels der zuvor beschriebenen Augenklappe 10 und einer davon entfernt angeordneten Datenverarbeitungseinrichtung 30 zur Verfügung gestellt. Die zur drahtlosen, bidirektionalen Kommunikation ausgebildete Datenschnittstelle 22 kann auf drahtlosem Wege mit der Datenverarbeitungseinrichtung 30 kommunizieren. Zu diesem Zweck empfängt die Datenverarbeitungseinrichtung 30 drahtlose Datensignale 32 von der Datenschnittstelle 22, die Teil der Sensoreinheit 14 der Augenklappe 10 ist. Die Datenverarbeitungseinrichtung umfasst wenigstens eine Recheneinheit 34 zur Datenverarbeitung, einen Bildschirm 36 zur Visualisierung der empfangenen und verarbeiteten Daten 32 und eine zur drahtlosen, bidirektionalen Kommunikation ausgebildete Datenschnittstelle 38, die mit der Datenschnittstelle 22 der Augenklappe 10 kommuniziert. Das erfindungsgemäße Verfahren sieht die folgenden Schritte vor: zunächst wird die Augenklappe 10 mitsamt der Sensoreinrichtung 14 am zu überwachenden Auge positioniert, wonach die Aktivierung der Sensoreinrichtung 14 erfolgen kann. Die von der Sensoreinrichtung 14 ermittelten Bewegungs- und/oder Lagedaten werden an die Datenverarbeitungseinrichtung 30 übermittelt; diese wertet die übertragenen Bewegungs- und/oder Lagedaten aus und visualisiert die ausgewerteten Bewegungs- und/oder Lagedaten. Wahlweise können die in der Datenspeichereinheit 26 gespeicherten Daten unabhängig von der zur drahtlosen, bidirektionalen Kommunikation ausgebildeten Datenschnittstelle 22, 38 an die Datenverarbeitungseinrichtung 30 übermittelt werden, bspw. durch Entnahme einer Speicherkarte aus der Augenklappe 10 und durch deren Positionierung in der Datenverarbeitungseinrichtung 30.

Die Bewegungs- und/oder Lagedaten können bei Bedarf innerhalb eines beliebigen Koordinatensystems auf wenigstens einen frei bestimmbaren Sollwert normiert werden. Außerdem kann vorgesehen sein, dass jede Abweichung der Bewegungs- und/oder Lagedaten vom wenigstens einen Sollwert wenigstens eine Aktion auslösen kann. Bei dieser Aktion kann es sich bspw. um einen visuellen und/oder akustischen und/oder durch wenigstens ein Vibrationssignal dargestellten Hinweis handeln.

Weiterhin kann optional eine Kalibrierung die in einer bestimmten Orientierung angeordneten Sensoreinheit 14 nach Anlegen der Augenklappe 10 und durch Bestimmung der Lage der Körperachse in Relation zur Sensoreinrichtung 14 vorgenommen werden, wodurch die Position der Augenklappe in Bezug auf die Kopfposition eindeutig bestimmt werden kann.

## Patentansprüche

1. Augenklappe (10) zur zumindest teilweisen oder partiellen Abdeckung eines menschlichen oder tierischen Auges und zu dessen Abschirmung vor mechanischen und/oder klimatischen Einflüssen und/oder vor elektromagnetischer Strahlung, insbesondere Lichtstrahlung, wobei die Augenklappe (10) wenigstens eine das Auge und dessen Umgebung zumindest partiell abdeckende Schale (12) aus biegesteifem Material sowie eine Sensoreinheit (14) aufweist, die zumindest einen Lage- und/oder Bewegungssensor (16) zur kontinuierlichen Erfassung von Bewegungs- und/oder Lagedaten der Augenklappe (10), einen Mikrocontroller (18) zur Verarbeitung der Ausgangssignale (20) des wenigstens einen Lage- und/oder Bewegungssensors (16), eine zur drahtlosen, bidirektionalen Kommunikation bzw. Datenübertragung ausgebildete Datenschnittstelle (22) sowie einen elektrischen Energiespeicher (24) zur elektrischen Energieversorgung zumindest des Mikrocontrollers (18) und der Datenschnittstelle (22) umfasst.

2. Augenklappe nach Anspruch 1, bei welcher der Sensoreinheit (14) eine dauerhafte und/oder temporär mit dem Lage- und/oder Bewegungssensor (16) koppelbare Datenspeichereinheit (26) zur Speicherung zumindest eines Teils der vom Sensor (16) erfassten Bewegungs- und/oder Lagedaten zugeordnet ist.

3. Augenklappe nach Anspruch 1 oder 2, bei der die Sensoreinheit (14) Mittel (28) zur Ausgabe eines akustischen Signals enthält.

4. Augenklappe nach einem der vorstehenden Ansprüche, wobei der Mikrocontroller (22) eine Signalquelle zur Erzeugung eines absoluten oder relativen Zeitindex enthält.

5. Augenklappe nach Anspruch 4, wobei Zeitindex und/oder Bewegungs- und/oder Lagedaten in der Datenspeichereinheit (26) speicherbar und/oder über die Datenschnittstelle (22) übertragbar sind.

6. Augenklappe nach einem der vorstehenden Ansprüche, wobei über die zur drahtlosen, bidirektionalen Kommunikation ausgebildete Datenschnittstelle (22) ein externer Zugriff auf die Sensoreinrichtung (16) erfolgt bzw. ermöglicht ist.

7. Augenklappe nach einem der vorstehenden Ansprüche, wobei wenigstens zwei der Bestandteile der Sensoreinheit (14) in einem Bauteil integriert sind.

8. Augenklappe nach einem der vorstehenden Ansprüche, wobei die Augenklappe (10) durch zwei Schalen (12) gebildet ist, zwischen denen wenigstens eines der Bestandteile der Sensoreinheit (14) angeordnet ist.

9. Verfahren zur kontinuierlichen Ermittlung von Bewegungs- und/oder Lagedaten eines menschlichen oder tierischen Kopfes mittels einer Augenklappe (10) und einer Datenverarbeitungseinrichtung (30), wobei die Augenklappe (10) wenigstens eine Sensoreinheit (14) mit mindestens einem Lage- und/oder Bewegungssensor (16), einen Mikrocontroller (18), einen Energiespeicher (24) und eine zur drahtlosen, bidirektionalen Kommunikation ausgebildete Datenschnittstelle (22) aufweist, und wobei die Datenverarbeitungseinrichtung (30) wenigstens eine Recheneinheit (34) zur Datenverarbeitung, einen Bildschirm (36) zur Visualisierung der verarbeiteten Daten (32) und eine zur drahtlosen, bidirektionalen Kommunikation ausgebildete Datenschnittstelle (38) umfasst, und wobei das Verfahren wenigstens die folgenden Schritte aufweist:
- Positionieren der Augenklappe (10) und der Sensoreinrichtung (14) am Auge;
- Aktivieren der Sensoreinrichtung (14);
- Übertragung der von der Sensoreinrichtung (14) ermittelten Bewegungs- und/oder Lagedaten an die Datenverarbeitungseinrichtung (30);
- Auswertung der übertragenen Bewegungs- und/oder Lagedaten in der Datenverarbeitungseinrichtung (30);
- Visualisierung und/oder Speicherung der ausgewerteten Bewegungs- und/oder Lagedaten.

10. Verfahren nach Anspruch 9, wobei die Sensoreinheit (14) mit einer dauerhaft und/oder temporär mit der Sensoreinheit (14) verbindbaren Datenspeichereinheit (26) kommuniziert, in welcher die Bewegungs- und/oder Lagedaten zusätzlich oder alternativ zur Datenübermittlung über die Datenschnittstelle (22) gespeichert werden können.

11. Verfahren nach Anspruch 10, wobei die Sensoreinheit (14) eine Signalquelle zur Erzeugung eines absoluten oder relativen Zeitindex enthält, welcher zusammen mit den Bewegungs- und/oder Lagedaten an die Datenverarbeitungseinrichtung (30) übermittelt und/oder in der Datenspeichereinheit (26) gespeichert wird.

12. Verfahren nach Anspruch 10 oder 11, wobei die in der Datenspeichereinheit (26) gespeicherten Daten unabhängig von der zur drahtlosen, bidirektionalen Kommunikation ausgebildeten Datenschnittstelle (22) an die Datenverarbeitungseinrichtung (30) übermittelt werden.

13. Verfahren nach einem der Ansprüche 9 bis 12, wobei die Bewegungs- und/oder Lagedaten innerhalb eines beliebigen Koordinatensystems auf wenigstens einen frei bestimmbaren Sollwert normiert werden können.

14. Verfahren nach Anspruch 13, wobei jede Abweichung der Bewegungs- und/oder Lagedaten vom wenigstens einen Sollwert wenigstens eine Aktion auslösen kann, und wobei es sich bei der wenigstens einen Aktion insbesondere um einen visuellen und/oder akustischen und/oder durch wenigstens ein Vibrationssignal dargestellten Hinweis handelt.

15. Verfahren nach einem der Ansprüche 9 bis 14, bei dem eine Kalibrierung der Sensoreinrichtung nach Anbringen der Augenklappe (10) durch Bestimmung der Lage der Körperachse in Relation zur Sensoreinrichtung (14) zur eindeutigen Bestimmung der Lage des Kopfes erfolgt.
